# FASCICULE DE BREVET EUROPEEN

(11) **EP 2 380 615 B1**
(45) Date de publication et mention de la délivrance du brevet: **28.11.2012**
(21) Numéro de dépôt: 11172223.7
(22) Date de dépôt: 29.06.2009
(51) Int. Cl.: A61M 3/02, F04B 43/12

(54) **Pompe péristaltique et ligne d'irrigation**
Peristaltische Pumpe und Irrigationsschlauch
Peristaltic pump and irrigation line

(30) Priorité: 03.07.2008 EP 08159641
(43) Date de publication de la demande: 26.10.2011
(62) Demande divisionnaire de: 09772402.5
(73) Titulaire: Bien-Air Holding SA, 2500 Bienne 6 (CH)
(72) Inventeur: Maître, Luc, 2885 Epauvilliers (CH); Ryser, Cyril, 2720 Tramelan (CH); Farine, Laurent, 2740 Moutier (CH); Siegenthaler, Didier, 2087 Cornaux (CH)
(74) Mandataire: Giraud, Eric

(56) Documents cités:
- EP-A- 1 400 691
- GB-A- 2 012 373
- US-A- 4 537 561
- US-A- 5 927 956

## Description

La présente invention concerne une ligne d'irrigation. Plus précisément, la présente invention concerne une ligne d'irrigation reliée à une poche de sérum physiologique.

Dans le domaine des interventions chirurgicales dans la cavité buccale et en particulier en implantologie dentaire, il est nécessaire de réaliser des perçages et des taraudages dans l'os de la mâchoire. Pour que ce travail puisse être effectué dans de bonnes conditions, il faut amener du sérum physiologique dans la région du champ opératoire. A cet effet, le praticien dispose de poches de sérum physiologique qui se vident par l'intermédiaire d'une ligne d'irrigation stérile. Cette ligne d'irrigation est classiquement passée sous les galets d'une pompe péristaltique qui assure une irrigation continue et à volume constant du champ opératoire. Lorsqu'une poche de sérum physiologique est vide ou entre deux patients successifs, le praticien doit retirer la ligne d'irrigation et la jeter avec la poche de sérum et renouveler l'opération d'installation d'une ligne d'irrigation reliée à une poche de sérum pleine.

On a remarqué qu'avec les pompes péristaltiques du commerce, l'opération qui consiste à placer la ligne d'irrigation sous les galets de ces pompes n'est pas aisée. Le praticien doit en effet faire usage de ses deux mains pour pouvoir déformer la ligne d'irrigation et la mettre dans une forme qui permette son installation sous les galets de la pompe. Il s'agit là d'une opération délicate qui requiert de la dextérité de la part du praticien et qui représente pour ce dernier une charge de travail supplémentaire. En outre, il n'est pas rare que le praticien ne parvienne pas à installer la ligne d'irrigation à la première tentative, de sorte qu'il est obligé de répéter ces opérations.

Le principe de base du fonctionnement d'une pompe péristaltique est simple. Lorsqu'un galet comprime la ligne d'irrigation, il provoque l'avancement d'un volume de fluide et crée en même temps une dépression qui permet l'arrivée du volume de fluide suivant. Les galets sont au nombre de trois. On comprend que lorsque ces galets tournent et viennent successivement comprimer la ligne d'irrigation, ils exercent sur celle-ci une force de traction tangente à la trajectoire circulaire desdits galets et orientée dans le même sens que le sens de rotation desdits galets. Il est donc nécessaire de prévoir des mesures pour immobiliser la ligne d'irrigation au niveau des galets de la pompe péristaltique. Le plus souvent, dans les pompes péristaltiques du commerce, la ligne d'irrigation est pincée entre deux mâchoires en amont et en aval des galets de la pompe. On comprend immédiatement l'inconvénient d'une telle solution: si la ligne d'irrigation n'est pas convenablement disposée et que les mâchoires se referment sur elle, la ligne d'irrigation va subir des contraintes de cisaillement et va percer.

Le document US 5,927,956 divulgue une portion de tube flexible pour une ligne d'irrigation qui omet de décrire une pièce support munie d'une pince de préhension comprenant deux mâchoires sur lesquelles il faut presser pour faire fléchir la plaque support.

La présente invention a pour but de remédier aux problèmes susmentionnés ainsi qu'à d'autres encore en procurant une ligne d'irrigation dont la mise en oeuvre est d'une très grande simplicité, ne requérant que des gestes simples de la part du praticien et évitant tout risque de percer ou de sectionner la ligne d'irrigation.

A cet effet, la présente invention concerne une portion de tube flexible d'une ligne d'irrigation selon la revendication 1 annexée à la présente demande de brevet.

Des modes de réalisation avantageux de la portion de tube flexible de la ligne d'irrigation conforme à la présente invention font l'objet des revendications dépendantes.

Grâce à ces caractéristiques, la présente invention procure une portion de tube flexible d'une ligne d'irrigation qui, de par sa longueur et la distance qui sépare ses deux extrémités, présente un profil qui lui permet de se positionner directement sous le chemin de roulement des galets d'une pompe péristaltique, sans que le praticien n'ait besoin de déformer le tube ou de le manipuler d'une quelconque autre manière pour pouvoir le monter sur la pompe. Le praticien gagne ainsi du temps et évite tout stress inutile qui pourrait avoir des conséquences négatives sur le déroulement de son intervention. En outre, les risques d'abîmer la ligne d'irrigation par exemple en la perçant, voire d'endommager la pompe sont pratiquement inexistants.

Le geste de montage de la portion de tube flexible est également simplifié au maximum. Il suffit en effet de déformer légèrement de manière élastique les moyens qui supportent la portion de tube flexible pour pouvoir monter cette dernière sur la pompe. Cette opération se fait avec deux doigts d'une seule main.

Puis, d'un simple geste de la part du praticien, la portion de tube est amenée sous le chemin de roulement des galets et finalement pressée contre ces derniers, se retrouvant ainsi en position de travail. En automatisant ainsi l'opération d'installation de la portion de tube, on décharge le praticien d'un travail qui, par le passé, pouvait s'avérer long et fastidieux et qui présentait des risques non négligeables de percer le tube ou d'endommager la pompe suite à un mauvais positionnement dudit tube.

D'autres caractéristiques et avantages de la présente invention ressortiront plus clairement de la description détaillée qui suit d'un mode de réalisation de la ligne d'irrigation selon l'invention, cet exemple étant donné à titre purement illustratif et non limitatif seulement en liaison avec le dessin annexé sur lequel :
- la figure 1 est une vue en perspective de la ligne d'irrigation ;
- la figure 2 est une vue en perspective du tiroir vide en position ouverte ;

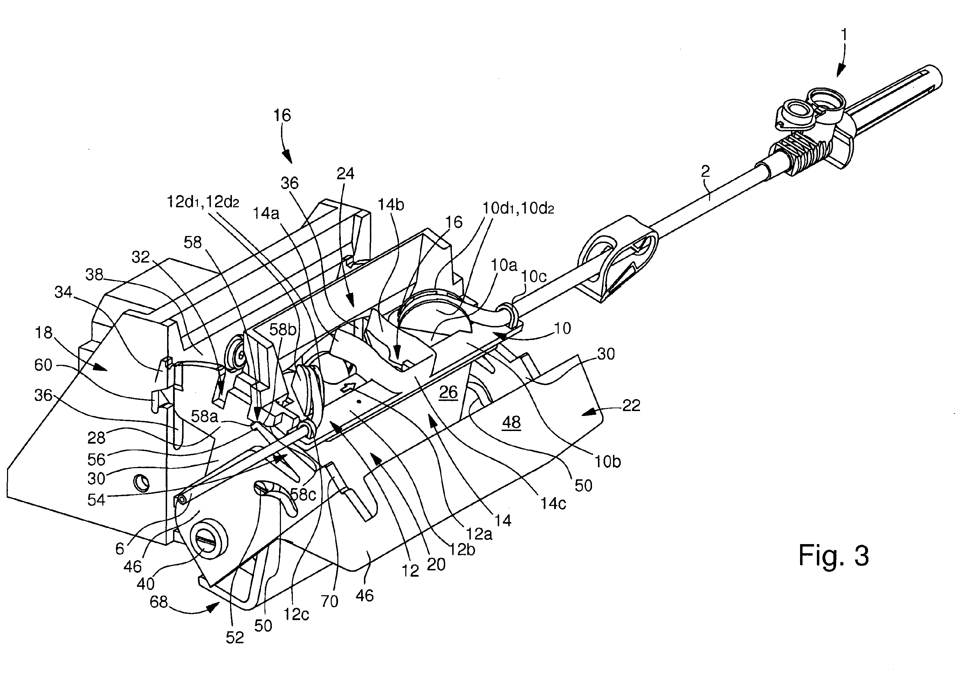

- la figure 3 est une vue en perspective du tiroir en position ouverte avec la ligne d'irrigation chargée dans le tiroir ;
- la figure 4a est une vue de face de la pompe péristaltique avec la ligne d'irrigation en place, le tiroir étant omis ;
- la figure 4b est une vue en perspective de la pompe péristaltique avec la ligne d'irrigation en place, le tiroir étant omis ;
- la figure 5a est une vue du tiroir de côté et en coupe, le tiroir étant complètement ouvert ;
- la figure 5b est une vue de côté de la pompe, le tiroir étant complètement ouvert ;
- la figure 6a est une vue du tiroir de côté et en coupe, le tiroir étant dans une première position intermédiaire ;
- la figure 6b est une vue de côté de la pompe, le tiroir étant dans une première position intermédiaire ;
- la figure 7a est une vue de côté et en coupe de la pompe, le tiroir étant dans une deuxième position intermédiaire ;
- la figure 7b est une vue de côté de la pompe, le tiroir étant dans une deuxième position intermédiaire ;
- la figure 8 est une vue de côté de la pompe, le tiroir étant fermé, et
- la figure 9 est une vue en perspective du tiroir en position fermée.

La présente invention concerne une ligne d'irrigation pour amener du sérum physiologique au niveau du champ opératoire. On s'intéressera tout d'abord à la ligne d'irrigation, la pompe péristaltique étant décrite dans un second temps.

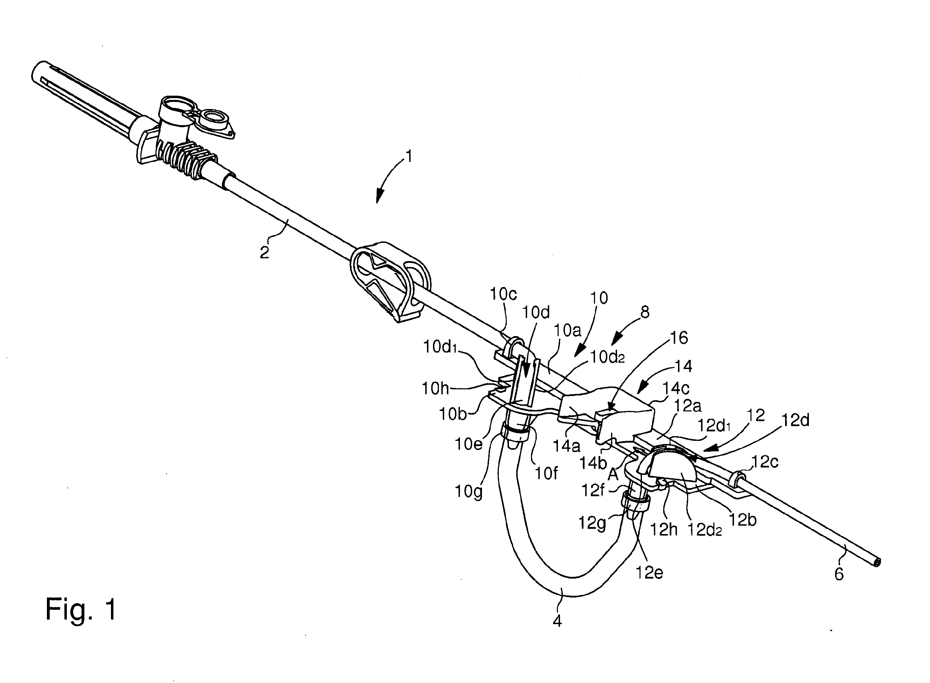

La ligne d'irrigation, désignée dans son ensemble par la référence numérique générale 1, comprend (voir figure 1) trois portions de tube, à savoir un tube d'admission 2 qui est relié à une poche (non représentée) de sérum physiologique et qui est prolongé successivement par un tube de pompage 4 et par un tube de sortie 6. Le tube d'admission 2 et le tube de sortie 6 sont typiquement réalisés en PVC. Quant au tube de pompage 4, il est préférentiellement réalisé en silicone, mais peut également être réalisé en PVC ou en un autre matériau souple et résistant.

Les galets de la pompe péristaltique qui sera décrite en détail ultérieurement roulent sur le tube de pompage 4. Lorsqu'à un moment donné, l'un des galets écrase le tube de pompage 4, il provoque l'avancement d'un volume de fluide en direction du tube de sortie 6 et crée en même temps une dépression qui permet l'arrivée d'un nouveau volume de fluide en provenance de la poche via le tube d'admission 2.

Le tube de pompage 4 est précédé par le tube d'admission 2 et suivi par le tube de sortie 6.

La ligne d'irrigation 1 selon l'invention est complétée par une pièce support 8. Cette pièce support 8 est de forme généralement rectiligne et présente deux bras étagés 10 et 12 qui sont reliés entre eux de manière élastique par une pince de préhension 14 en forme de U et dotée de deux mâchoires 14a et 14b. Les deux mâchoires 14a, 14b de la pince de préhension 14 sont reliées entre elles par une paroi de fond 14c qui assure également la liaison entre les deux bras étagés 10 et 12. On notera que l'élasticité des deux bras étagés 10 et 12 est obtenue par l'enlèvement de matière visible en 16 entre les deux mâchoires 14a, 14b de la pince de préhension 14.

Chacun des deux bras étagés 10 et 12 présente la même structure formée par une plaque de base 10a, 12a qui s'étend en avant et à un niveau inférieur de celui d'un longeron 10b, 12b. Ainsi, le longeron 10b du bras étagé 10 présente vers son extrémité libre un anneau 10c sous lequel est passé le tube d'admission 2. Le tube d'admission 2 est ensuite passé dans une rainure de guidage 10d en arc de cercle délimitée par deux parois verticales 10d₁ et 10d₂ avant de pénétrer dans un trou 10e dans lequel il peut être éventuellement collé au moyen d'une colle biocompatible stérilisable ou fixé par toute autre méthode telle que, par exemple, le soudage aux ultrasons. Le trou 10e débouche sous la surface inférieure de la plaque de base 10a dans un embout cranté 10f sur lequel l'une des extrémités du tube de pompage 4 est forcée puis sertie au moyen d'une bague de sertissage 10g que l'on peut déplacer manuellement pour venir coiffer ladite extrémité du tube de pompage 4.

De manière analogue, l'autre extrémité libre du tube de pompage 4 est forcée sur un embout cranté 12f et sertie au moyen d'une bague de sertissage 12g. L'embout cranté 12f débouche sur la face supérieure de la plaque de base 12a dans un trou 12e dans lequel est engagée et éventuellement collée ou encore soudée aux ultrasons l'une des extrémités libres du tube de sortie 6. Le tube de sortie 6 est ensuite passé dans une rainure de guidage 12d en arc de cercle délimitée par deux parois verticales 12d₁ et 12d₂ avant d'être glissé sous un anneau 12c qui se dresse à l'extrémité libre du longeron 12b.

A son extrémité restée libre, le tube d'admission 2 est relié à une poche (non représentée) de liquide physiologique. Quant au tube de sortie 6, il est relié à son extrémité libre à un instrument dentaire ou chirurgical.

On appréciera dès maintenant que la longueur du tube de pompage 4 et la distance séparant les deux embouts crantés 10f et 12f sont calculées de telle manière que ledit tube de pompage 4 adopte spontanément une forme qui le rend apte à être glissé sous les galets de la pompe péristaltique.

On note également la présence, sur chacune des plaques de base 10a, 12a des deux bras étagés 10 et 12, d'un ergot de verrouillage respectivement 10h, 12h qui permet le verrouillage de la pièce support 8 sur le bâti de la pompe péristaltique comme décrit en détail ci-après.

On comprendra aussi qu'en raison de l'élasticité de la pièce support 8, les deux ergots de verrouillage 10h, 12h ont tendance à se rapprocher l'un de l'autre lorsque l'utilisateur serre entre deux de ses doigts les mâchoires 14a, 14b de la pince de préhension 14. Deux flèches, repérées A sur le dessin, invitent d'ailleurs l'utilisateur à exercer une pression sur les mâchoires 14a, 14b de la pince 14.

On notera enfin que la ligne d'irrigation est, bien entendu, livrée complètement assemblée à son utilisateur. Il suffira donc à ce dernier d'introduire la partie tube de pompage de la ligne d'irrigation dans le bâti de la pompe péristaltique, puis de relier la ligne d'une part à la poche de fluide physiologique, et d'autre part à l'instrument chirurgical ou dentaire qu'il doit utiliser.

On s'intéresse maintenant à la pompe péristaltique.

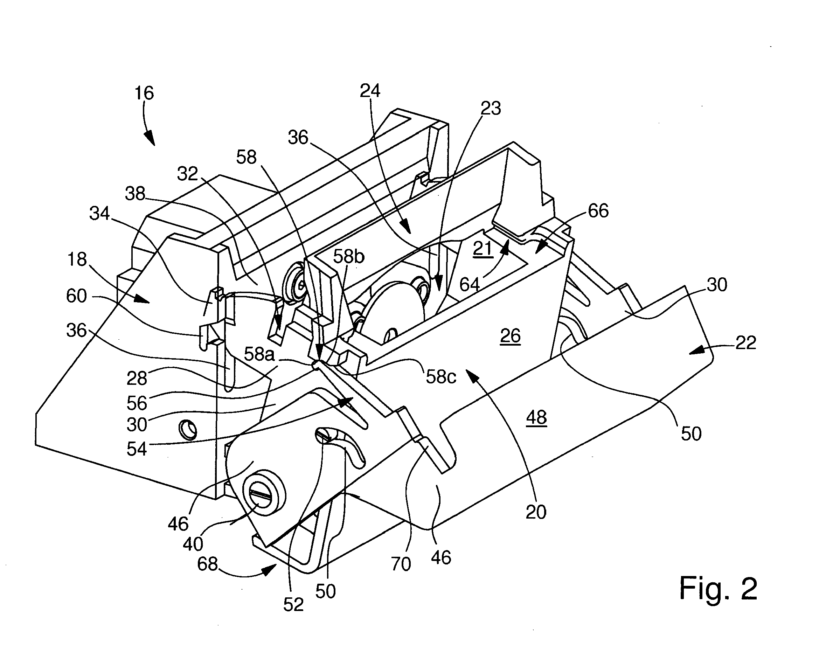

Désignée dans son ensemble par la référence numérique générale 16, la pompe péristaltique comprend essentiellement (voir figure 2) un bâti 18, un tiroir 20 et une poignée 22. Sur la figure 2, on a représenté la pompe péristaltique 16 avec son tiroir 20 vide en position ouverte. On remarque d'emblée que le tiroir 20 présente une forme générale extérieure parallélépipédique et définit avec une paroi intérieure 21 sensiblement en forme d'arc de cercle un logement 23 pour la réception de la ligne d'irrigation 1 et plus précisément de la pièce support 8 et du tube de pompage 4. Le tiroir 20 est prolongé vers le haut par une plaque d'arrêt 24 qui s'étend parallèlement à et en retrait par rapport à la face avant 26 du tiroir 20. Au moins un et préférentiellement deux crochets 28 sont prévus dans le prolongement des petits côtés 30 du tiroir 20, au niveau de l'extrémité supérieure de ces dits petits côtés 30. On remarque que les crochets 28 présentent chacun une rainure verticale 32 qui les sépare en partie des petits côtés 30 du tiroir 20 et qu'ils se terminent par une dent 34 qui a pour fonction de limiter le débattement dudit tiroir 20 lorsqu'on ouvre celui-ci. En effet, comme on le verra ci-après, le tiroir 20 effectue essentiellement un mouvement de pivotement au cours duquel les crochets 28 glissent dans deux fentes verticales 36 ménagées dans la grande face 38 du bâti 18 de façon que, lorsque le tiroir 20 est complètement ouvert, il est retenu par les dents 34 des crochets 28 qui viennent en prise avec la grande face 38 du bâti 18.

Le tiroir 20 et la poignée 22 sont montés pivotants sur le bâti 18 de la pompe 16 autour de deux axes de pivotement communs 40 situés de part et d'autre de l'ensemble formé par le tiroir 20 et la poignée 22. On remarque que le tiroir 20, la poignée 22 et le bâti 18 de la pompe 16 sur lequel le tiroir 20 et la poignée 22 sont montés forment un ensemble constructif indépendant et amovible. Ainsi, en cas de défaillance du moteur de la pompe 16 par exemple, il suffit d'effectuer un échange standard entre l'ensemble défectueux et un nouvel ensemble. De même, l'ensemble constructif amovible peut être installé soit dans un boîtier prévu spécialement à cet effet soit, par exemple, dans une unité d'alimentation et de contrôle couramment appelée « unit » que l'on trouve chez tous les praticiens.

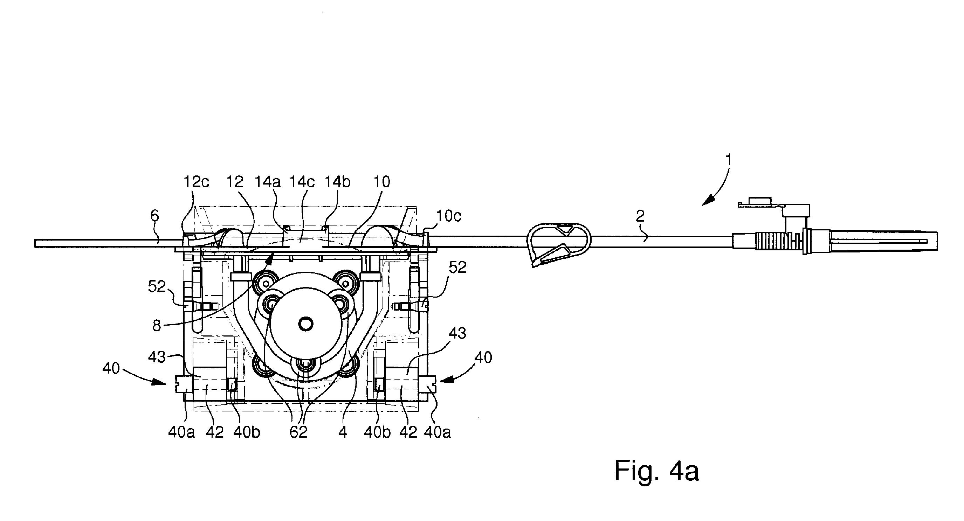

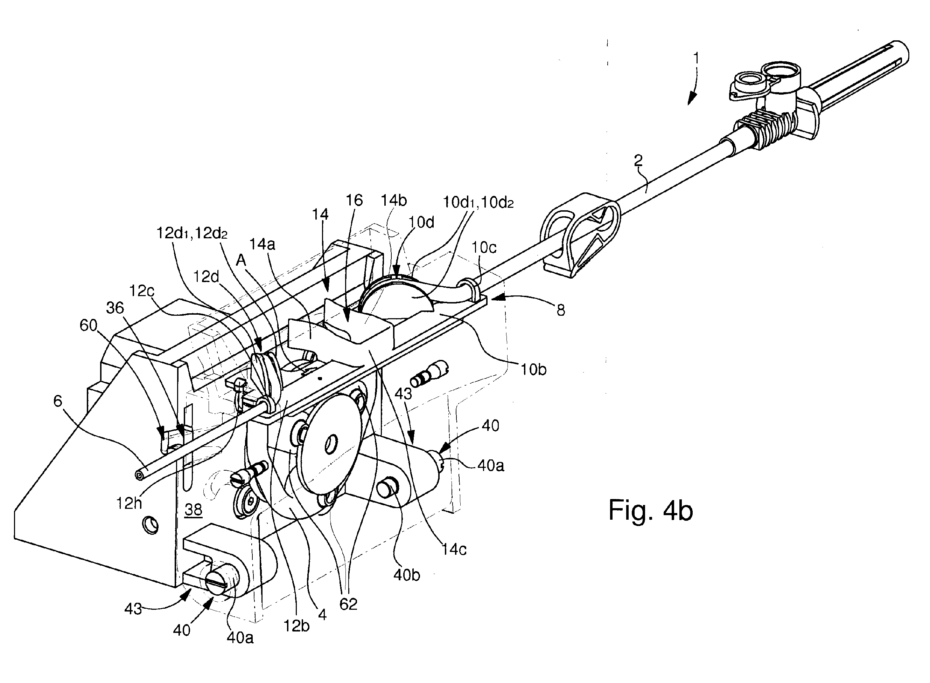

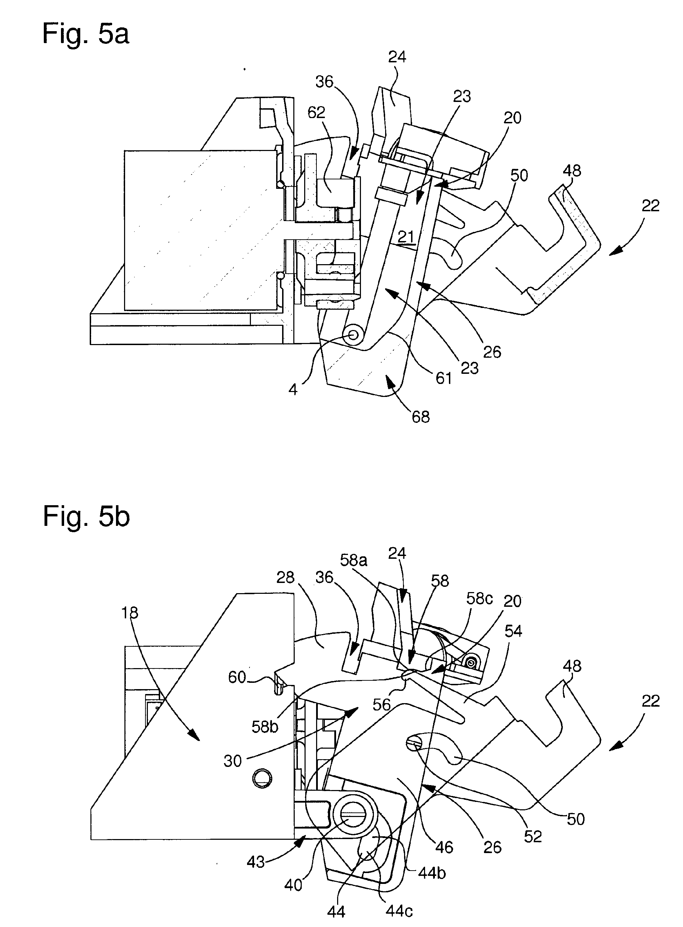

Chaque axe de pivotement 40 débouche de part et d'autre d'un trou traversant 42 pratiqué dans une nervure 43 qui s'étend perpendiculairement à la grande face 38 du bâti 18 (voir figures 4a et 4b). A sa première extrémité libre 40a, l'axe de pivotement 40 sert de support au tiroir 20 par l'intermédiaire de la poignée 22. A sa seconde extrémité libre 40b, l'axe de pivotement 40 suit le profil d'un chemin de came 44 ménagé sur la face extérieure de la paroi intérieure 21 du tiroir 20 (voir figures 5a et 5b).

La poignée 22 se compose pour l'essentiel de deux parois verticales 46 parallèles entre elles et reliées l'une à l'autre par une barre d'actionnement 48. On remarque que chacune des parois 46 de la poignée 22 présente un chemin de came 50 en forme de banane qui est suivi par un ergot 52 fixé par tout moyen approprié dans le petit côté 30 du tiroir 20.

On remarque enfin que la poignée 22 présente deux bras inclinés 54 qui s'étendent parallèlement aux petits côtés 30 du tiroir 20 et qui se terminent chacun par un ergot 56. Comme on le verra ci-dessous, ces deux bras inclinés 54 coopèrent alternativement avec un chemin de came 58 prévu à l'extrémité supérieure du petit côté 30 du tiroir 20 et avec une encoche 60 ménagée sur les côtés de la grande face 38 du bâti 18. On notera que le chemin de came 58 présente deux plans parallèles et inclinés 58a et 58c reliés entre eux par un plan horizontal 58b.

On s'intéresse maintenant à l'opération de chargement de la ligne d'irrigation 1 et de fermeture du tiroir 20. On qualifiera de positif les mouvements qui tendent à éloigner le tiroir 20 et la poignée 22 du bâti 18 de la pompe 16, et de négatif les mouvements qui tendent à rapprocher le tiroir 20 et la poignée 22 du bâti 18 de la pompe 16.

L'opération de mise en place de la ligne d'irrigation 1 commence par l'introduction du tube de pompage 4 et de la pièce support 8 dans le logement 23 du tiroir 20 (voir figure 5a). La paroi intérieure 21 a une forme qui suit sensiblement le profil du tube de pompage 4. Le tube de pompage 4 est prépositionné dans le logement 23 par une came de guidage 61 prévue dans le fond dudit logement 23 et qui oriente le tube de pompage 4 en direction des galets 62 de la pompe péristaltique 16 (voir figure 5a). La pièce support 8 est introduite dans le logement 23 avec le côté ouvert des deux mâchoires 14a, 14b de la pince 14 orienté du côté des galets 62 de la pompe 16.

Comme déjà mentionné ci-dessus, pour permettre une installation facile de la pièce support 8 dans le logement 23 du tiroir, l'utilisateur a juste à serrer avec deux de ses doigts les deux mâchoires 14a, 14b de la pince 14. De par leur élasticité, les deux bras étagés 10, 12 vont fléchir et les ergots de verrouillage 10h, 12h vont se rapprocher l'un de l'autre, ce qui va permettre de les présenter en face d'une rainure 64 ménagée à la base de la plaque d'arrêt 24 (voir figure 2). Lorsque l'utilisateur relâche la pression sur les deux mâchoires 14a, 14b de la pince 14, les ergots de verrouillage 10h, 12h vont par leur face inclinée venir faire saillie dans la rainure 64, assurant le maintien de la pièce support 8 dans le tiroir 20.

Ainsi engagée, la pièce support 8 repose sur le rebord 66 du tiroir 20 (voir figure 2) par les plaques de base 10a, 12a de ses bras 10 et 12. L'utilisateur a donc juste besoin de glisser la partie tube de pompage 4 de la ligne d'irrigation 1 dans le logement 23 du tiroir 20 et d'exercer une légère pression sur les mâchoires 14a, 14b de la pince 14 pour mettre en place la pièce support 8 dans ledit tiroir 20. Il s'agit là d'une opération extrêmement simple et quasi intuitive qui ne nécessite que l'usage d'une seule main. En outre, à aucun moment l'utilisateur n'a besoin d'ajuster le profil du tuyau de pompage 4 pour que celui-ci s'adapte au profil du chemin de roulement des galets 62 de la pompe 16. L'utilisateur gagne ainsi un temps précieux, évite tout stress inutile et ne risque pas d'endommager la ligne d'irrigation, voire la pompe elle-même en raison d'un mauvais positionnement du tube de pompage relativement aux galets de ladite pompe.

Lorsque le tube de pompage 4 et sa pièce support 8 sont installés dans le logement 23 du tiroir 20, on peut commencer à fermer ce dernier (voir figures 5a et 5b). A cet effet, on exerce sur la barre d'actionnement 48 de la poignée 22 une poussée dans le sens négatif, c'est-à-dire en direction du bâti 18 de la pompe 16. La poignée 22 commence alors à pivoter autour de l'axe de pivotement 40, entraînant avec elle le tiroir 20 dans le même mouvement de pivotement autour dudit axe 40. En effet, à ce stade du mouvement, le tiroir 20 et la poignée 22 sont couplés par le biais des deux bras inclinés 54 de la poignée 22 qui butent par leurs ergots 56 contre les plans inclinés 58a des chemins de came 58. Le tiroir est également empêché de monter par le glissement des crochets 28 dans les rainures 36.

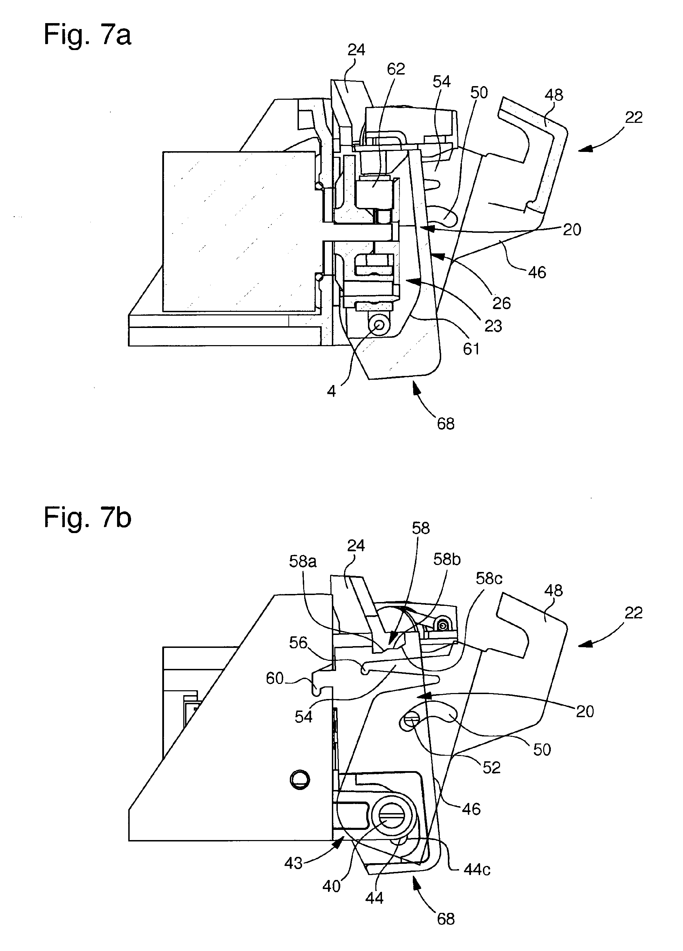

La poignée 22 entraîne donc le tiroir 20 en pivotement négatif autour de l'axe 40 en direction du bâti 18 de la pompe 16 jusqu'à ce que la plaque d'arrêt 24 du tiroir 20 vienne buter contre la partie supérieure dudit bâti 18. A ce moment là (voir figures 7a et 7b), les ergots 56 des bras inclinés 54 échappent aux plans inclinés 58c des chemins de came 58 et la poignée 22 et le tiroir 20 ne sont plus couplés en pivotement.

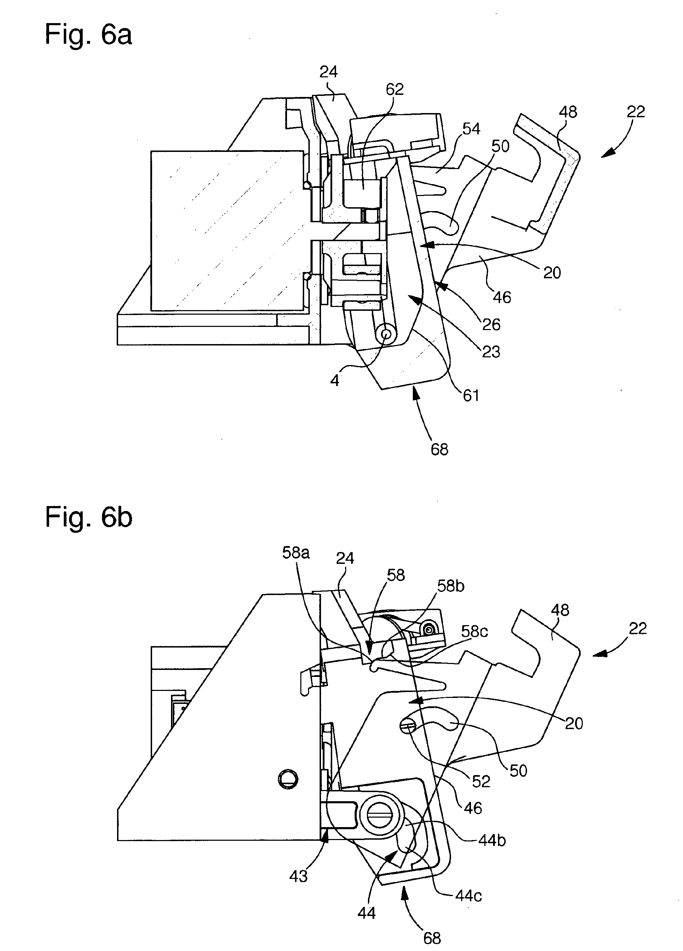

Quand on continue de pousser la poignée 22 vers le bâti 18 de la pompe 16 par action sur la barre d'actionnement 48, la poignée 22 continue son mouvement de pivotement autour de son axe 40, tandis que le tiroir 20 effectue un mouvement combiné de pivotement et de translation vers le haut. On remarque en effet (voir figures 6a et 6b) qu'au moment où la poignée 22 et le tiroir 20 se découplent, le tiroir 20 est dans une position inclinée avec sa base 68 qui est écartée du bâti 18 de la pompe 16. Il faut bien comprendre que la base 68 du tiroir 20 est sensiblement au même niveau que le tube de pompage 4 au fond de son logement 23. Par conséquent, dans cette position où le tiroir 20 est incliné, le tube de pompage 4 est encore dégagé du chemin de roulement des galets 62 de la pompe 16, ce qui facilite l'opération de fermeture du tiroir 20.

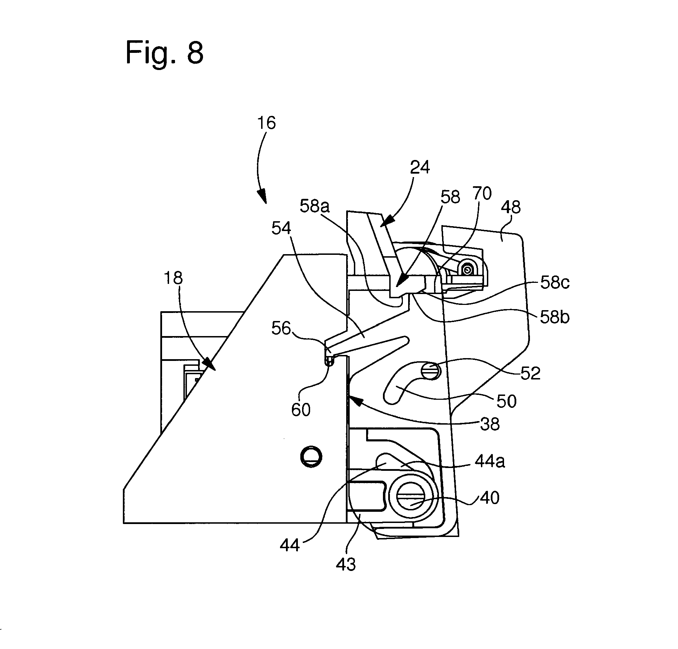

Néanmoins, il faut ensuite pouvoir amener le tube de pompage 4 sous le chemin de roulement des galets 62, puis forcer ce tube 4 contre ces mêmes galets 62. Ceci est réalisé grâce au double mouvement de pivotement et de translation vers le haut du tiroir 20. En effet, au cours du mouvement de pivotement de la poignée 22, le tiroir 20 pivote également et est guidé dans son déplacement par le chemin de came 44 qui se déplace relativement à la seconde extrémité libre 40b de l'axe de pivotement 40. Ce chemin de came 44 présente deux portions rectilignes 44a et 44c reliées entre elles par une portion coudée 44b. Au début du mouvement de pivotement du tiroir 20 (figure 5a), l'axe de pivotement 40 se trouve au fond de la première portion rectiligne 44a du chemin de came 44. A la fin du mouvement de pivotement du tiroir 20 (figure 8), l'axe de pivotement 40 se retrouve au fond de la seconde portion rectiligne 44c dudit chemin de came 44. Ce déplacement relatif du chemin de came 44 par rapport à l'axe de pivotement 40 du tiroir 20 permet de ramener la base 68 de ce dernier en appui contre le bâti 18 de la pompe 16. Simultanément, l'ergot 52 fixé sur le tiroir 20 se déplace du bas vers le haut dans le chemin de came 50 ménagé dans la poignée 22. Ce mouvement permet d'achever la phase de fermeture du tiroir 20 en forçant le tiroir 20 à effectuer un mouvement de translation verticale de bas en haut. Ainsi, en ramenant la base 68 du tiroir 20 en appui contre le bâti 18 de la pompe 16 durant la première phase de montée du tiroir 20, on amène le tube de pompage 4 sous le chemin de roulement des galets 62, tandis que le déplacement en translation verticale vers le haut de ce même tiroir 20 permet de forcer ledit tube de pompage 4 contre lesdits galets 62.

Le verrouillage final du tiroir 20 (voir figure 8) est réalisé par les ergots 56 des bras inclinés 54 qui viennent se loger dans les encoches 60 ménagées sur le bâti 18 de la pompe 16, ceci afin d'éviter que le tiroir 20 ne s'ouvre sous l'effet des vibrations causées par ces galets 62. On voit également que la poignée 22 présente un épaulement 70 qui, en position fermée de la poignée 22, vient se loger sous la surface plane 58b du chemin de came 58 afin de maintenir le tiroir 20 dans sa position haute et de supporter les forces radiales des galets 62. Enfin, la grande face 38 du bâti 18 vient faire saillie dans les rainures verticales 32 des crochets 28 pour assurer le verrouillage du tiroir 20 et de la poignée 22 en pivotement.

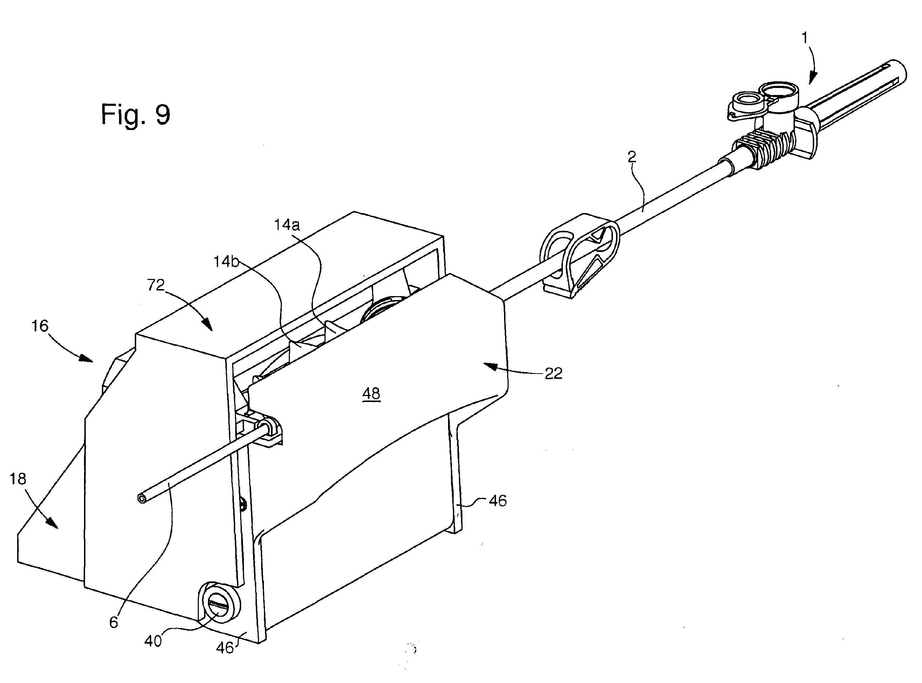

La pompe est représentée en position fermée à la figure 9, un capot 72 coiffant l'ensemble.

Le déverrouillage du tiroir 20 s'effectue dans l'ordre inverse des étapes décrites ci-dessus en liaison avec la fermeture du tiroir 20. On commence tout d'abord par exercer une traction dans le sens positif sur la barre d'actionnement 48 de la poignée 22. La poignée 22 commence alors à pivoter autour de son axe 40, ce qui provoque la sortie des ergots 56 des encoches 60 et l'effacement de l'épaulement 70 de dessous la surface plane 58b du chemin de came 58. Simultanément, l'ergot 52 fixé sur le tiroir 20 se déplace de haut en bas dans le chemin de came 50 ménagé dans la poignée 22, ce qui force le tiroir 20 à effectuer un mouvement de translation verticale du haut vers le bas. Ce mouvement permet d'éloigner le tube de pompage 4 du chemin de roulement des galets 62 en abaissant dans un premier temps le tube de pompage 4 par rapport au niveau des galets 62. Parallèlement, le chemin de came 44 se déplace relativement à la seconde extrémité libre 40b de l'axe de pivotement 40. Au début du mouvement de pivotement du tiroir 20, l'axe de pivotement 40 se trouve au fond de la seconde portion rectiligne 44c du chemin de came 44. A la fin du mouvement de pivotement du tiroir 20, l'axe de pivotement 40 se retrouve au fond de la première portion rectiligne 44a dudit chemin de came 44. Ce déplacement relatif du chemin de came 44 par rapport à l'axe de pivotement 40 du tiroir 20 permet de faire pivoter la base 68 du tiroir 20 dans le sens positif en l'éloignant du bâti 18 de la pompe 16 et donc d'écarter le tube de pompage 4 du chemin de roulement des galets 62.

Arrivée à un certain point de son pivotement, la poignée 22 se couple avec le tiroir 20 par le biais des ergots 56 de ses bras inclinés 54 qui viennent en prise avec les plans incliné 58c des chemins de came 58. La poignée 22 entraîne donc le tiroir 20 en pivotement positif autour de l'axe de pivotement 40 en l'éloignant encore davantage du bâti 18 de la pompe 16, grâce à quoi le tube de pompage 4 et la pièce support 8 sont complètement dégagés dudit bâti 18. On notera que le pivotement du tiroir 20 est arrêté lorsque les dents 34 des crochets 28 viennent en prise avec la grande face 38 du bâti 18. On notera par ailleurs que lors du mouvement de descente verticale du tiroir 20, les rainures verticales 32 se dégagent de l'emprise de la grande face 38 du bâti 18. Il suffit alors d'exercer une pression sur les mâchoires 14a, 14b de la pince 14 pour dégager les ergots de verrouillage 10h, 12h des rainures 64 et pour pouvoir retirer le tube de pompage 4 et sa pièce support 8 du tiroir 20.

## Revendications

1. Portion de tube flexible pour une ligne d'irrigation (1) reliée à une réserve de fluide à usage dentaire ou chirurgical, la portion de tube flexible (4) étant destinée à coopérer avec les galets (62) d'une pompe péristaltique (16) pour la distribution du fluide, la portion de tube flexible étant montée sur une pièce support (8) également flexible et comprenant des moyens (10f, 12f) qui définissent entre ses deux extrémités libres une distance qui, combinée à sa longueur, confèrent à la portion de tube flexible un profil déterminé lui permettant de se positionner directement sous le chemin de roulement des galets (62) de la pompe (16), la portion de tube flexible étant **caractérisée en ce que** la pièce support (8) est munie d'une pince de préhension (14) comprenant deux mâchoires (14a, 14b) sur lesquelles il faut presser pour faire fléchir la pièce support (8).

2. Portion de tube flexible selon la revendication 1, **caractérisée en ce que** la pièce support (8) est munie de deux embouts (10f, 12f) sur lesquels sont engagées les extrémités libres de la portion de tube flexible.

3. Portion de tube flexible selon la revendication 2, **caractérisée en ce que** la longueur de la portion de tube flexible (4) et la distance séparant les deux embouts (10f, 12f) sont calculées de telle manière que ladite portion de tube flexible (4) adopte spontanément une forme qui la rend apte à être glissée sous les galets (62) de la pompe péristaltique (16).

4. Portion de tube flexible selon l'une quelconque des revendications 2 ou 3, **caractérisée en ce que** la pièce support (8) comprend des moyens (10h, 12h, 64) pour son maintien sur le bâti (18) de la pompe (16).

5. Portion de tube flexible selon l'une quelconque des revendications 1 à 4, **caractérisée en ce que** la pièce support (8) comprend des moyens pour la connexion de la portion de tube flexible (4) d'une part à un tube d'admission (2) par lequel arrive le fluide et d'autre part à un tube de sortie (6) par lequel le fluide est expulsé par la pompe (16).

## Claims

1. Flexible tube portion for an irrigation line (1) connected to a supply of fluid for surgical or dental use, the flexible tube portion (4) being intended to cooperate with the rollers (62) of the peristaltic pump (16) for distributing the fluid, the flexible tube portion being mounted on a support part (8) which is also flexible and including means (10f, 12f) that define between the two free ends of said tube portion, a distance, which, combined with the length thereof, gives said flexible tube portion a determined profile enabling it to be positioned directly underneath the raceway of the rollers (62) of the pump (16), the flexible tube portion being **characterized in that** the support part (8) is fitted with a gripping clamp (14) including two jaws (14a, 14b) which have to be pressed to bend the support part (8).

2. Flexible tube portion according to claim 2, **characterized in that** the support part (8) is provided with two tips (10f, 12f) on which the free ends of the flexible tube portion are engaged.

3. Flexible tube portion according to claim 2, **characterized in that** the length of the flexible tube portion (4) and the distance separating the two tips (10f and 12f) are calculated such that said flexible tube portion (4) spontaneously adopts a shape enabling said tube portion to slide under the rollers (62) of the peristaltic pump (16).

4. Flexible tube portion according to any of claims 2 or 3, **characterized in that** the support part (8) includes means (10h, 12h, 64) for holding said part on the frame (18) of the pump (16).

5. Flexible tube portion according to any of claims 1 to 4, **characterized in that** the support part (8) includes means for connecting the flexible tube portion (4) both to an inlet tube (2) through which the fluid arrives and to an outlet tube (6) through which the fluid is expelled by the pump (16).

## Patentansprüche

1. Flexibler Schlauchabschnitt für eine Spülleitung (1), die mit einem Fluidvorrat zur dentalen oder chirurgischen Verwendung verbunden ist, wobei der flexible Schlauchabschnitt (4) dazu vorgesehen ist, mit Rollen (62) einer Peristaltikpumpe (16) zur Verteilung des Fluids zusammenzuwirken, wobei der flexible Schlauchabschnitt (4) auf einem gleichermaßen flexiblen Trägerteil (8) montiert ist, welches Mittel (10f, 12f) aufweist, die zwischen ihren beiden freien Enden einen Abstand definieren, der kombiniert mit seiner Länge dem flexiblen Schlauchabschnitt ein vorgegebenes Profil verleiht, das es ihm erlaubt, ihn direkt unter der Rollenbahn der Rollen (62) der Pumpe (16) zu positionieren, wobei der flexible Schlauchabschnitt **dadurch gekennzeichnet ist, dass** er mit einer Griffklemme (14) versehen ist, die zwei Backen (14a, 14b) aufweist, auf welche zum Entspannen der Trägerteil (8) zu drücken ist.

2. Flexibler Schlauchabschnitt nach Anspruch 1, **dadurch gekennzeichnet, dass** das Trägerteil (8) mit zwei Ansatzstücken (10f, 12f) versehen ist, an welchen die freien Enden des flexiblen Schlauchstücks angebunden sind.

3. Flexibler Schlauchabschnitt nach Anspruch 2, **dadurch gekennzeichnet**, das die Länge des flexiblen Schlauchstücks (4) und der die beiden Ansatzstücke (10f, 12f) trennende Abstand derart berechnet sind, dass der genannte Schlauchabschnitt (4) spontan eine Form annimmt, die ihn dazu befähigt, unter die Rollen (62) der Peristaltikpumpe (16) zu rutschen.

4. Flexibles Schlauchstück nach einem beliebigen der Ansprüche 2 oder 3, **dadurch gekennzeichnet, dass** das Trägerteil (8) Mittel (10h, 12h, 64) zu seinem Verbleib auf dem Gestell (18) der Pumpe (16) aufweist.

5. Flexibler Schlauchabschnitt nach einem beliebigen der Ansprüche 1 bis 4, **dadurch gekennzeichnet, dass** das Trägerteil (8) Mittel aufweist, um das flexible Schlauchstück (4) einerseits mit einem Aufnahmeschlauch (2), über welchen das Fluid ankommt, und andererseits mit einem Ausgangsschlauch (6), über welchen das Fluid über die Pumpe (16) ausgestoßen wird, zu verbinden.
